# EUROPEAN PATENT APPLICATION

(11) **EP 4 360 655 A2**
(43) Date of publication of application: **01.05.2024**
(21) Application number: 24158150.3
(22) Date of filing: 31.07.2020
(51) Int. Cl.: A61K 47/64

(54) **MOLECULAR GUIDE SYSTEM PEPTIDES AND USES THEREOF**

(30) Priority: 01.10.2019 US 201962908687 P
(62) Divisional of application: 20871090.5
(71) Applicant: SRI International, Menlo Park, CA 94025 (US)
(72) Inventor: BROWN, Kathlynn C., California 94025 (US); MCGUIRE, Miguel, California 94025 (US); VENUGOPAL, Indu, California 94025 (US); ALLRED, Curtis, California 94025 (US)
(74) Representative: D Young & Co LLP

(57) **Abstract**

Disclosed are compositions comprising a nucleic acid sequence conjugated to one or more MGS peptides. Disclosed are methods of modifying gene expression of a gene of interest comprising administering to a cell a composition comprising a nucleic acid sequence conjugated to one or more MGS peptides, wherein the nucleic acid sequence binds to the RNA transcribed from the gene of interest, the gene of interest or a sequence upstream of the gene of interest. Disclosed are methods of targeting a gene of interest in an intracellular target comprising administering to a cell a composition comprising a nucleic acid sequence conjugated to one or more MGS peptides, wherein the MGS peptide targets the intracellular target, wherein the nucleic acid sequence binds to the RNA transcribed from the gene interest in an intracellular target. Disclosed are methods of treating a subject in need thereof comprising administering to the subject in need thereof an effective amount of a nucleic acid sequence conjugated to one or more MGS peptides, wherein the MGS peptide targets an intracellular target involved in a disease process.

## Description

### CROSS-REFERENCE TO RELATED APPLICATIONS

This application claims the benefit of U.S. Provisional Patent Application No. 62/908,687, filed on October 1, 2019, which is incorporated by reference herein in its entirety.

### STATEMENT REGARDING FEDERALLY SPONSORED RESEARCH

This invention was made with government support under the contract number W81XWH-16-1-0262 awarded by USA Medical Research Acquisition Activity (USAMRAA), and under grant numbers 7R01CA164447 and 7R01EB014244 awarded by The National Institutes of Health. The government has certain rights to this invention.

### REFERENCE TO SEQUENCE LISTING

The Sequence Listing submitted July 31, 2020 as a text file named "37794_0094P1_Sequence_Listing.txt," created on July 30, 2020, and having a size of 18,505 bytes is hereby incorporated by reference pursuant to 37 C.F.R. § 1.52(e)(5).

### BACKGROUND

There is need for systematic cell-targeting systems that deliver therapeutic nucleic acids to a specific cell type and to specific locations within that cell.

The ability to deliver a therapeutic to a specific target cell, and to the right compartment within that cell, can have numerous societal health benefits. Molecular Guide System (MGS) combinations can create a new class of safe and efficacious intracellular acting therapeutics with the potential to treat heretofore "undruggable" targets. This opens up new therapeutic strategies for the treatment of emerging infectious diseases and bioterrorism, novel therapies for cancer, diabetes, neurological and neurodegenerative diseases and even genetically inherited diseases. MGS combination therapeutics can create an entirely new market impacting virtually every disease state and creating a major breakthrough in modern medicine.

### BRIEF SUMMARY

Disclosed are compositions comprising a nucleic acid sequence conjugated to one or more MGS peptides.

Disclosed are methods of modifying gene expression of a gene of interest comprising administering to a cell a composition comprising a nucleic acid sequence conjugated to one or more MGS peptides, wherein the nucleic acid sequence binds to the RNA transcribed from the gene of interest, the gene of interest or a sequence upstream of the gene of interest.

Disclosed are methods of targeting a gene of interest in an intracellular target comprising administering to a cell a composition comprising a nucleic acid sequence conjugated to one or more MGS peptides, wherein the MGS peptide targets the intracellular target, wherein the nucleic acid sequence binds to the RNA transcribed from the gene interest in an intracellular target.

Disclosed are methods of treating a subject in need thereof comprising administering to the subject in need thereof an effective amount of a nucleic acid sequence conjugated to one or more MGS peptides, wherein the MGS peptide targets an intracellular target involved in a disease process.

Additional advantages of the disclosed method and compositions will be set forth in part in the description which follows, and in part will be understood from the description, or may be learned by practice of the disclosed method and compositions. The advantages of the disclosed method and compositions will be realized and attained by means of the elements and combinations particularly pointed out in the appended claims. It is to be understood that both the foregoing general description and the following detailed description are exemplary and explanatory only and are not restrictive of the invention as claimed.

### BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.

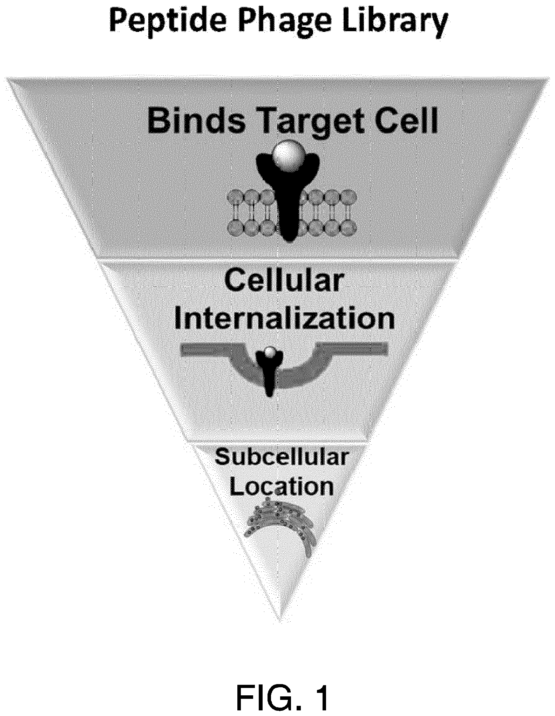

Figure 1 is a schematic diagram of the Molecular Guidance Systems (MGSs). The molecular targeting peptide will be identified by the FOX-Three technology. The peptide will deliver the cargo specifically to the target cell while avoiding uptake in other cells. Upon internalization, the peptide will direct the cargo to the desired location within the cell.
Figure 2 is a diagram showing examples of cell processes that can be manipulated by targeting therapeutic nucleic acid sequences to specific subcellular organelles.

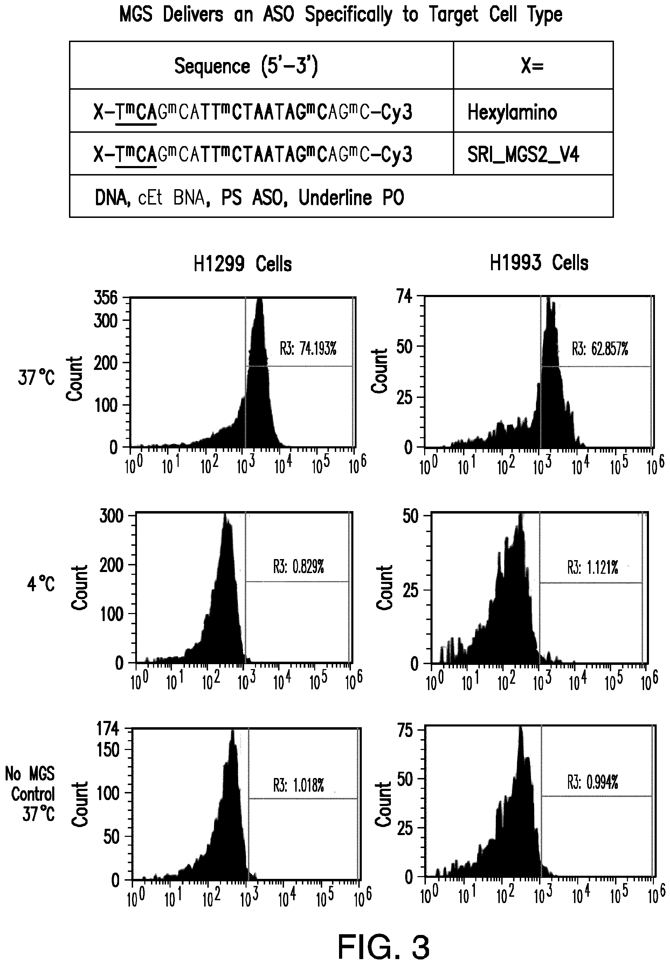

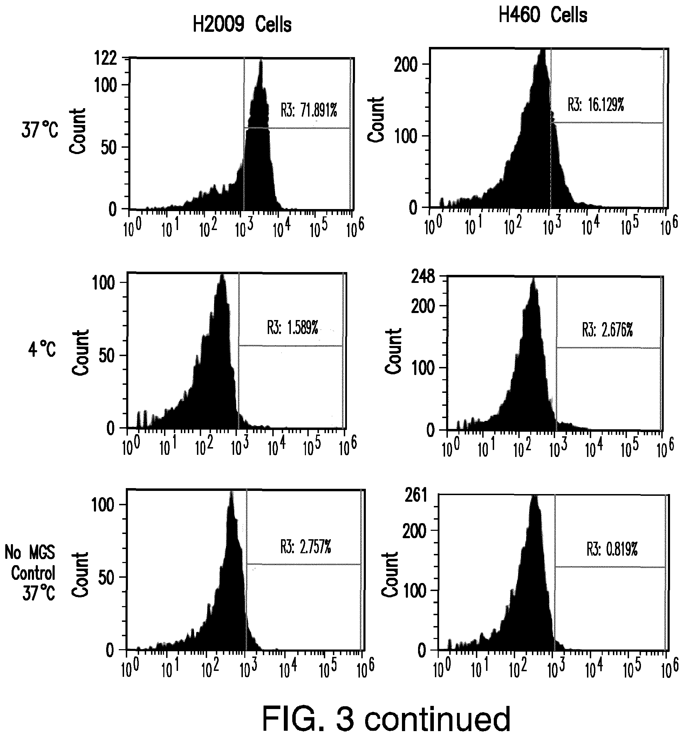

Figure 3 shows an example of an MGS with a fluorophore labeled nucleic acid sequence cargo is internalized into four different cells types at two different temperatures compared to the fluorophore labeled nucleic acid sequence's internalization profile without the aid of the MGS. T^{m}CAG^{m}CATT^{m}CTAATAG^{m}CAG^{m}C-Cy3 (SEQ ID NO:X) is the nucleic acid sequence used either conjugated to hexylamino or an MGS, wherein "m" represents a methylation. cEt-BNA is constrained ethyl bridged nucleic acid; PS ASO is phosphorothioate antisense oligonucleotides; PO: Phosphodiester linkages. The nucleotides in positions 4-6 and 17-19 are cEt BNA, black have phosphorothioate linkages and black/underlined have phosphodiester linkages.
Figure 4 shows actual fluorescence data of the experiment described in FIG.3 showing internalization of the fluorophore into the different cell types.
Figure 5 shows siRNA delivery into different cell types with and without being conjugated to an MGS.

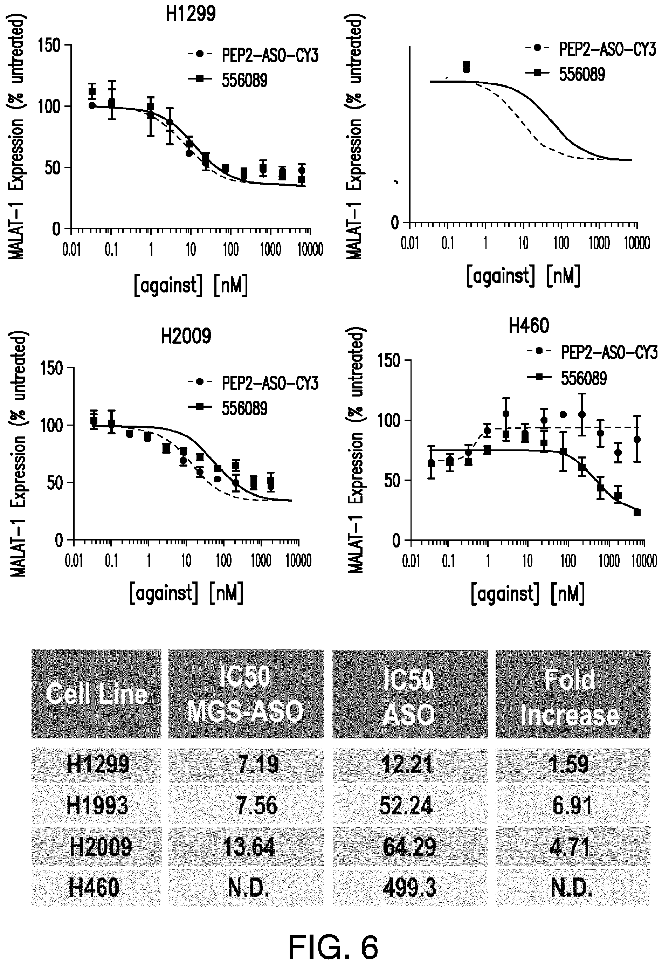

Figure 6 shows MGS delivery increases potency of antisense oligonucleotide (ASO) in target cells and reduces effects in non-target cells.

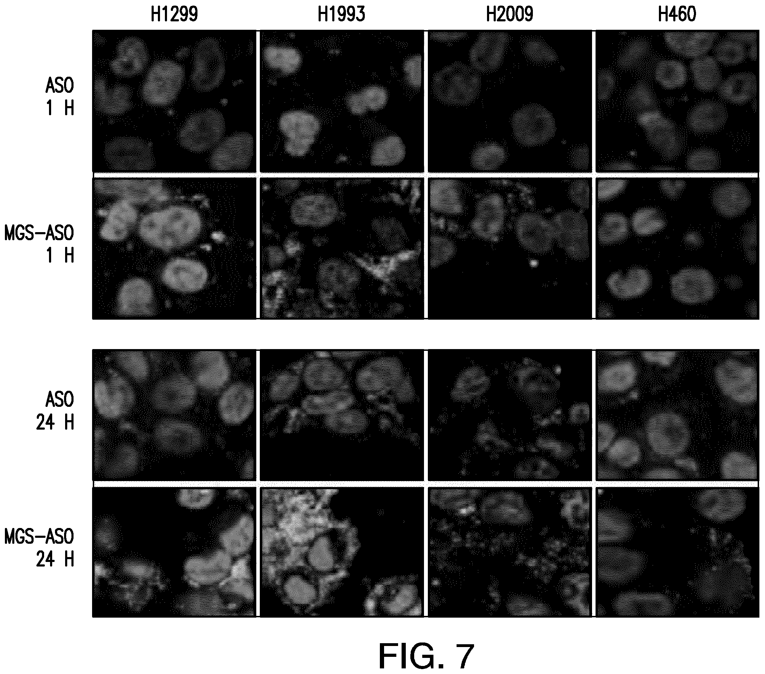

Figure 7 shows immunofluorescence studies where MGS improves ASO delivery to target cell types.

### DETAILED DESCRIPTION

The disclosed method and compositions may be understood more readily by reference to the following detailed description of particular embodiments and the Example included therein and to the Figures and their previous and following description.

It is to be understood that the disclosed method and compositions are not limited to specific synthetic methods, specific analytical techniques, or to particular reagents unless otherwise specified, and, as such, may vary. It is also to be understood that the terminology used herein is for the purpose of describing particular embodiments only and is not intended to be limiting.

Disclosed are materials, compositions, and components that can be used for, can be used in conjunction with, can be used in preparation for, or are products of the disclosed method and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a composition is disclosed and discussed and a number of modifications that can be made to a number of molecules including the MGS peptide are discussed, each and every combination and permutation of the compositions and the modifications that are possible are specifically contemplated unless specifically indicated to the contrary. Thus, if a class of molecules A, B, and C are disclosed as well as a class of molecules D, E, and F and an example of a combination molecule, A-D is disclosed, then even if each is not individually recited, each is individually and collectively contemplated. Thus, is this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

### A. Definitions

It is understood that the disclosed method and compositions are not limited to the particular methodology, protocols, and reagents described as these may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a ", "an", and "the" include plural reference unless the context clearly dictates otherwise. Thus, for example, reference to "a MGS" includes a plurality of such MGSs, reference to "the nucleic acid sequence" is a reference to one or more nucleic acid sequences and equivalents thereof known to those skilled in the art, and so forth.

As used herein, "treat" is meant to mean administer a composition of the invention to a subject, such as a human or other mammal (for example, an animal model), that has a disease or condition, in order to prevent or delay a worsening of the effects of the disease or condition, or to partially or fully reverse the effects of the disease or condition. In some aspects, the disease or condition can be cancer. Treatment may be administered to a subject who does not exhibit signs of a disease, disorder, and/or condition and/or to a subject who exhibits only early signs of a disease, disorder, and/or condition for the purpose of decreasing the risk of developing pathology associated with the disease, disorder, and/or condition. In some embodiments, treatment comprises delivery of one or more of the disclosed compositions to a subject.

As used herein, "prevent" is meant to mean minimize the chance that a subject who has an increased susceptibility for developing disease, disorder or condition will develop the disease, disorder or condition.

As used herein, the term "subject" refers to the target of administration, e.g., a human. Thus the subject of the disclosed methods can be a vertebrate, such as a mammal, a fish, a bird, a reptile, or an amphibian. The term "subject" also includes domesticated animals (e.g., cats, dogs, etc.), livestock (e.g., cattle, horses, pigs, sheep, goats, etc.), and laboratory animals (e.g., mouse, rabbit, rat, guinea pig, fruit fly, etc.). In one aspect, a subject is a mammal. In another aspect, a subject is a human. The term does not denote a particular age or sex. Thus, adult, child, adolescent and newborn subjects, as well as fetuses, whether male or female, are intended to be covered.

As used herein, the term "patient" refers to a subject afflicted with a disease or disorder. The term "patient" includes human and veterinary subjects. In some aspects of the disclosed methods, the "patient" has been diagnosed with a need for treatment prior to the administering step.

As used herein, the term "amino acid sequence" refers to a list of abbreviations, letters, characters or words representing amino acid residues. The amino acid abbreviations used herein are conventional one letter codes for the amino acids and are expressed as follows: A, alanine; C, cysteine; D aspartic acid; E, glutamic acid; F, phenylalanine; G, glycine; H histidine; I isoleucine; K, lysine; L, leucine; M, methionine; N, asparagine; P, proline; Q, glutamine; R, arginine; S, serine; T, threonine; V, valine; W, tryptophan; Y, tyrosine.

Polypeptide" as used herein refers to any peptide, oligopeptide, polypeptide, gene product, expression product, or protein. A polypeptide is comprised of consecutive amino acids. The term "polypeptide" encompasses naturally occurring or synthetic molecules.

In addition, as used herein, the term "polypeptide" refers to amino acids joined to each other by peptide bonds or modified peptide bonds, e.g., peptide isosteres, etc. and may contain modified amino acids other than the 20 gene-encoded amino acids. The polypeptides can be modified by either natural processes, such as post-translational processing, or by chemical modification techniques which are well known in the art. Modifications can occur anywhere in the polypeptide, including the peptide backbone, the amino acid side-chains and the amino or carboxyl termini. The same type of modification can be present in the same or varying degrees at several sites in a given polypeptide. Also, a given polypeptide can have many types of modifications. Modifications include, without limitation, acetylation, acylation, ADP-ribosylation, amidation, covalent cross-linking or cyclization, covalent attachment of flavin, covalent attachment of a heme moiety, covalent attachment of a nucleotide or nucleotide derivative, covalent attachment of a lipid or lipid derivative, covalent attachment of a phosphytidylinositol, disulfide bond formation, demethylation, formation of cysteine or pyroglutamate, formylation, gamma-carboxylation, glycosylation, GPI anchor formation, hydroxylation, iodination, methylation, myristolyation, oxidation, pergylation, proteolytic processing, phosphorylation, prenylation, racemization, selenoylation, sulfation, and transfer-RNA mediated addition of amino acids to protein such as arginylation. *(See* Proteins-Structure and Molecular Properties 2nd Ed., T.E. Creighton, W.H. Freeman and Company, New York (1993); Posttranslational Covalent Modification of Proteins, B.C. Johnson, Ed., Academic Press, New York, pp. 1-12 (1983)).

The phrase "nucleic acid sequence" as used herein refers to a naturally occurring or synthetic oligonucleotide or polynucleotide, whether DNA or RNA or DNA-RNA hybrid, single-stranded or double-stranded, sense or antisense, which is capable of hybridization to a complementary nucleic acid by Watson-Crick base-pairing. Nucleic acid sequences of the invention can also include nucleotide analogs (e.g., BrdU), and non-phosphodiester internucleoside linkages (*e.g.,* peptide nucleic acid (PNA) or thiodiester linkages). In particular, nucleic acid sequences can include, without limitation, DNA, RNA, cDNA, gDNA, ssDNA, dsDNA or any combination thereof.

As used herein, "effective amount" of a composition is meant to mean a sufficient amount of the composition to provide the desired effect. The exact amount required will vary from subject to subject, depending on the species, age, and general condition of the subject, the severity of disease (or underlying genetic defect) that is being treated, the particular compound used, its mode of administration, and the like. Thus, it is not possible to specify an exact "effective amount." However, an appropriate "effective amount" may be determined by one of ordinary skill in the art using only routine experimentation.

As used herein, "selectively binds" is meant that a nucleic acid sequence (e.g. cargo) or MGS recognizes and physically interacts with its target (for example, a specific cell type) and does not significantly recognize and interact with other targets.

The term "percent (%) homology" is used interchangeably herein with the term "percent (%) identity" and refers to the level of nucleic acid or amino acid sequence identity when aligned with a wild type sequence or sequence of interest using a sequence alignment program. For example, as used herein, 80% homology means the same thing as 80% sequence identity determined by a defined algorithm, and accordingly a homologue of a given sequence has greater than 80% sequence identity over a length of the given sequence. Exemplary levels of sequence identity include, but are not limited to, 80, 85, 90, 95, 98% or more sequence identity to a given sequence, e.g., any of the MGS sequences, as described herein. Exemplary computer programs which can be used to determine identity between two sequences include, but are not limited to, the suite of BLAST programs, e.g., BLASTN, BLASTX, and TBLASTX, BLASTP and TBLASTN, publicly available on the Internet. See also, Altschul, et al., 1990 and Altschul, et al., 1997. Sequence searches are typically carried out using the BLASTN program when evaluating a given nucleic acid sequence relative to nucleic acid sequences in the GenBank DNA Sequences and other public databases. The BLASTX program is preferred for searching nucleic acid sequences that have been translated in all reading frames against amino acid sequences in the GenBank Protein Sequences and other public databases. Both BLASTN and BLASTX are run using default parameters of an open gap penalty of 11.0, and an extended gap penalty of 1.0, and utilize the BLOSUM-62matrix. (See, e.g., Altschul, S. F., et al., Nucleic Acids Res.25:3389-3402, 1997.) A preferred alignment of selected sequences in order to determine "% identity" between two or more sequences, is performed using for example, the CLUSTAL-W program in Mac Vector version 13.0.7, operated with default parameters, including an open gap penalty of 10.0, an extended gap penalty of 0.1, and a BLOSUM 30 similarity matrix.

Substitutions, deletions, insertions or any combination thereof may be used to arrive at a final derivative, variant, or analog. Generally, these changes are done on a few nucleotides to minimize the alteration of the molecule. However, larger changes may be tolerated in certain circumstances.

Generally, the nucleotide identity between individual variant sequences can be at least 90%, 91%, 92%, 93%, 94%, 95%, 96%, 97%, 98%, 99%, or 100%. Thus, a "variant sequence" can be one with the specified identity to the parent or reference sequence (e.g. wild-type sequence) of the invention, and shares biological function, including, but not limited to, at least 80%, 81%, 82%, 83%, 84%, 85%, 86%, 87%, 88%, 89%, 90%, 91%, 92%, 93%, 94%, 95%, 96%, 97%, 98%, or 99% of the specificity and/or activity of the parent sequence. For example, a "variant sequence" can be a sequence that contains 1, 2, or 3, 4 nucleotide base changes as compared to the parent or reference sequence of the invention, and shares or improves biological function, specificity and/or activity of the parent sequence. Thus, a "variant sequence" can be one with the specified identity to the parent sequence of the invention, and shares biological function, including, but not limited to, at least 80%, 81%, 82%, 83%, 84%, 85%, 86%, 87%, 88%, 89%, 90%, 91%, 92%, 93%, 94%, 95%, 96%, 97%, 98%, or 99% of the specificity and/or activity of the parent sequence. The variant sequence can also share at least 80%, 81%, 82%, 83%, 84%, 85%, 86%, 87%, 88%, 89%, 90%, 91%, 92%, 93%, 94%, 95%, 96%, 97%, 98%, or 99% of the specificity and/or activity of a reference sequence (e.g. a MGS sequence).

As used herein, "modulate" is meant to mean to alter, by increasing or decreasing.

"Optional" or "optionally" means that the subsequently described event, circumstance, or material may or may not occur or be present, and that the description includes instances where the event, circumstance, or material occurs or is present and instances where it does not occur or is not present.

Ranges may be expressed herein as from "about" one particular value, and/or to "about" another particular value. When such a range is expressed, also specifically contemplated and considered disclosed is the range from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint, and independently of the other endpoint unless the context specifically indicates otherwise. Finally, it should be understood that all of the individual values and sub-ranges of values contained within an explicitly disclosed range are also specifically contemplated and should be considered disclosed unless the context specifically indicates otherwise. The foregoing applies regardless of whether in particular cases some or all of these embodiments are explicitly disclosed.

Unless defined otherwise, all technical and scientific terms used herein have the same meanings as commonly understood by one of skill in the art to which the disclosed method and compositions belong. Although any methods and materials similar or equivalent to those described herein can be used in the practice or testing of the present method and compositions, the particularly useful methods, devices, and materials are as described. Publications cited herein and the material for which they are cited are hereby specifically incorporated by reference. Nothing herein is to be construed as an admission that the present invention is not entitled to antedate such disclosure by virtue of prior invention. No admission is made that any reference constitutes prior art. The discussion of references states what their authors assert, and applicants reserve the right to challenge the accuracy and pertinency of the cited documents. It will be clearly understood that, although a number of publications are referred to herein, such reference does not constitute an admission that any of these documents forms part of the common general knowledge in the art.

Throughout the description and claims of this specification, the word "comprise" and variations of the word, such as "comprising" and "comprises," means "including but not limited to," and is not intended to exclude, for example, other additives, components, integers or steps. In particular, in methods stated as comprising one or more steps or operations it is specifically contemplated that each step comprises what is listed (unless that step includes a limiting term such as "consisting of'), meaning that each step is not intended to exclude, for example, other additives, components, integers or steps that are not listed in the step.

### B. Compositions

Disclosed are compositions comprising a cargo conjugated to one or more MGS peptides. In some aspects, the cargo is a nucleic acid. Thus, disclosed are compositions comprising a nucleic acid sequence conjugated to one or more MGS peptides. In some aspects, the disclosed compositions can be referred to membrane-permeable compositions or conjugates as they can be used for transport across a lipid membrane.

Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein.

### 1. MGS peptides

Disclosed herein are MGS peptides or targeting peptides. These peptides can selectively bind to cells. Examples of MGS peptides that can be used or modified in the disclosed compositions can include, but are not limited to, one or more of the MGS peptides disclosed in McGuire et al., Sci Rep. 2014 Mar 27; 4:4480. Examples of MGS peptides that can also be used in the disclosed compositions and methods, include, but are not limited to the MGS sequences shown in Table 1.

**Table 1. MGS peptide sequences.**

| **Peptide Name** | **Peptide Sequence** | **SRI REFERENCE NUMBER:** | **SEQ ID NO:** |
|---|---|---|---|
| MGS_H2009.1 | RGDLATLRQLAQEDGVVGVR | 44 | 1 |
| | D-Leu-RGDLATLRQL | 12 | 2 |
| | CH₃CO- D-Leu-RGDLATLRQL | 13 | 3 |
| MGS_2009.1 V4 | CH3CO-RGDLATLRQL-PEG₁₁-YC-NH₂ | 80 | 4 |
| | CH3CO-RGDLATLRQL-PEG₁₁-Y-NH₂ | | 5 |
| MGS_H2009.1 V5 | CH3CO-d(Leu)-RGDLATLRQL-PEG11-YC- | 81 | 6 |
| | NH2 | | |
| | CH3CO-d(Leu)-RGDLATLRQL-PEG₁₁-Y-NH₂ | | 7 |
| | CH₃CO-RGDLATLRQL | New # | 8 |
| MGS_1299.2 | YAAWPASGAWTGTAPCSAGT | 41 | 9 |
| | YAAWPASGAWT | 9 | 10 |
| | CHsCO-YAAWPASGAWT | 10 | 11 |
| MGS_1299.2 V4 | CH3CO-YAAWPASGAWT-PEG₁₁-C-NH₂ | 78 | 12 |
| | | | |
| MGS_HCC15.1 | ATEPRKQYATPRVFWTDAPG | 49 | 13 |
| | KQYATPRVFWT | 38 | 14 |
| | CH₃CO- KQYATPRVFWT | 39 | 15 |
| MGS_HCC15.1 V4 | CH₃CO- KQYATPRVFWT-PEG₁₁-C-NH₂ | 82 | 16 |
| | | | |
| MGS_HCC15.2 | FHAVPQSFYTAP | 50 | 17 |
| | CH₃CO-FHAVPQSFYT | 6 | 18 |
| MGS_HCC15.2 V8 | CH3CO-FHAVPQSFYT-PEG₁₁-C-NH₂ | 83 | 19 |
| | | | |
| MGS_1299.3 | LQWRRDDNVHNFGVWARYRL | 42 | 20 |
| | LQWRRNFGVWARYRL | 36 | 21 |
| MGS_1299.3 V2 | CH3CO-LQWRRNFGVWARYRL-PEG11-C- NH2 | 79 | 22 |
| | | | |
| MGS_H460.1 | EAMNSAEQSAAVVQWEKRRI | 51 | 23 |
| MGS_H1299.1 | VSQTMRQTAVPLLWFWTGSL | 40 | 24 |
| MGS_A549.1 | MTVCNASQRQAHAQATAVSL | 52 | 25 |
| MGS_HCC95.1 | MRGQTGKLPTEHFTDTGVAF | 60 | 26 |
| MGS_H1155.1 | MTGKAAAPHQEDRHANGLEQ | 61 | 27 |
| MGS_H1155.2 | MEKLPLSKTGRTVSEGVSPP | 62 | 28 |
| MGS_H666.1 | TNSCRGDWLCDAVPEKARV | 63 | 29 |
| | | | |
| MGS_H2009.2 | EHPWFNMWSWATQVQE | 45 | 30 |
| | EHPWFNMW SWATQVQEKKK | 27 | 31 |
| MGS_H2009.3 | YPGSPTQYPSSMHEYHSSSE | 46 | 32 |
| MGS_H2009.4 | AHTIDDEWASYHMQQWNSPP | 47 | 33 |
| MGS_H2009.5 | FEEFYSRQSNTIPYPQQYKG | 48 | 34 |
| | | | |
| MGS_H1993.1 | SVEYWGERMYYDVMESLGFS | 54 | 35 |
| MGS_H1993.2 | FAAKRAEWWDPGQLWDAVWN | 55 | 36 |
| MGS_H1993.3 | QEALEEWFWKMMPWSGPSGQ | 56 | 37 |
| MGS_H1993.4 | TWTDFGQWPWPFGAEGTRAF | 57 | 38 |
| MGS_H1993.5 | MDGATWWTQLDPLLVWEGET | 58 | 39 |
| MGS_H1993.6 | SADWFQGPAEWLLEGWMGPL | 59 | 40 |
| | | | |
| MGS_MCF7.1 | LTVHGRGPEYNPSWNRRAFP | 53 | 41 |
| MGS_A20.1 | SAKTAVSQRVWLPSHRGGEP | 64 | 42 |
| MGS_A20.2 | KSREHVNNSACPSKRITAAL | 65 | 43 |
| MGS_PCM.1 | WLSEAGPVVTVRALRGTGSW | 66 | 44 |
| MGS_C2C12.1 | TGGETSGIKKAPYASTTRNR | 67 | 45 |
| MGS_C2C12.2 | SHHGVAGVDLGGGADFKSIA | 68 | 46 |
| MGS_C2C12.3 | SNSPLGLKDEATQRLVLEQAKWLA | 69 | 47 |
| MGS_XS52.1 | GPEDTSRAPENQQKTFHRRW | 70 | 48 |
| MGS_XS52.2 | SGETGSNLVGHELDFRPGSPSP | 71 | 49 |
| MGS_XS106.1 | RYSPAATAEGRSVSKELLRV | 72 | 50 |
| MGS_717US.1 | GQELGAWTRSKGPEVQTSVL | 73 | 51 |
| MGS_7175.1 | ASTWRGTSAGGNRLEKMEVT | 74 | 52 |
| MGS_RIP.1 | LSGTPERSGQAVKVKLKAIP | 75 | 53 |
| MGS_RIP.2 | GAWEAVRDRIAEWGSWGIPS | 76 | 54 |
| MGS_MArg.1_Ba cterial | AMDMYSIEDRYFGGYAPEVG | 77 | 55 |

In some aspects, the one or more MGS peptides comprise the sequence of YAAWPASGAWTGTAPCSAGT (SEQ ID NO:9); LQWRRDDNVHNFGVWARYRL (SEQ ID NO:20); RGDLATLRQLAQEDGVVGVR (SEQ ID NO:1); ATEPRKQYATPRVFWTDAPG (SEQ ID NO:13); FHAVPQSFYTAP (SEQ ID NO:17); EHPWFNMWSWATQVQE (SEQ ID NO:30) or combinations thereof.

In some aspects, the one or more MGS peptides have a sequence identity of at least 90, 91, 92, 93, 94, 95, 96, 97, 98, or 99% identity with YAAWPASGAWTGTAPCSAGT (SEQ ID NO:9); LQWRRDDNVHNFGVWARYRL (SEQ ID NO:20); RGDLATLRQLAQEDGVVGVR (SEQ ID NO:1); ATEPRKQYATPRVFWTDAPG (SEQ ID NO:13); FHAVPQSFYTAP (SEQ ID NO:17); or EHPWFNMWSWATQVQE (SEQ ID NO:30). In some aspects, the one or more MGS peptides have a sequence identity of at least 90, 91, 92, 93, 94, 95, 96, 97, 98, or 99% identity with any of the MGS peptides disclosed herein.

In some aspects, MGS peptides can be optimized. For example, SEQ ID NOs:3, 4, 5, 6, 7, 8, 11, 12, 15, 16, 18, 19, and 22 are optimized. Optimized peptides were obtained by applying modifications to the individual parental peptide sequence identified by the FOX-3 platform technology. These modifications are used to identify the essential amino acids within the parental sequence that are required for cell-specific binding and internalization. These modifications are obtained by a combination of alanine scanning and truncations of the amino-terminal region and c-terminal region of the parental peptide. PEG11 provides protection of the C-terminus of the MGS peptide, provides a spacer between the peptide and the cargo molecule attached through the cysteine at the C-terminus, and enhances solubility of the MGS-peptide. Modification at the amino-terminus by acetylation (CH3CO-) and/or d-amino acids, such as d(Leu) protect against degradation by peptidases in blood. There is not a uniform length of optimized peptide that can be applied to all MGS peptides and all changes need to be tested to confirm the effect on peptide uptake and stability. In peptide 73, the YC is used to allow us to monitor peptide synthesis and concentration by the absorbance of light at 280 nm. Without the addition of tyrosine (Y), this peptide would be significantly more difficult to monitor. Disclosed are modified peptides comprising the sequence of CH₃CO-YAAWPASGAWT-PEG₁₁-C-NH₂ (SEQ ID NO:12), CH3CO-LQWRRNFGVWARYRL-PEG₁₁-C-NH₂ (SEQ ID NO:22), CH3CO-RGDLATLRQL-PEG₁₁-YC-NH₂ (SEQ ID NO:4), CH3CO-RGDLATLRQL-PEG₁₁-Y-NH₂ (SEQ ID NO:5), CH₃CO-d(Leu)-RGDLATLRQL-PEG₁₁-YC-NH₂ (SEQ ID NO:6), CH₃CO-d(Leu)-RGDLATLRQL-PEG₁₁-Y-NH₂ (SEQ ID NO:7), CH₃CO-KQYATPRVFWT -PEG₁₁-C-NH₂ (SEQ ID NO:16), or CH₃CO-FHAVPQSFYT-PEG₁₁-C-NH₂ (SEQ ID NO:19). Thus, in some aspects, the one or more MGS peptides of the disclosed compositions can be CH₃CO-YAAWPASGAWT-PEG₁₁-C-NH₂ (SEQ ID NO:12), CH3CO-LQWRRNFGVWARYRL-PEG₁₁-C-NH₂ (SEQ ID NO:22), CH3CO-RGDLATLRQL-PEG₁₁-YC-NH₂ (SEQ ID NO:4), CH3CO-RGDLATLRQL-PEG₁₁-Y-NH₂ (SEQ ID NO:5), CH₃COd(Leu)-RGDLATLRQL-PEG₁₁-YC-NH₂ (SEQ ID NO:6), CH₃CO-d(Leu)-RGDLATLRQL-PEG₁₁-Y-NH₂ (SEQ ID NO:7), CH₃CO- KQYATPRVFWT -PEG₁₁-C-NH₂ (SEQ ID NO:16), or CH₃CO-FHAVPQSFYT-PEG₁₁-C-NH₂ (SEQ ID NO:19) or a combination thereof.

In some aspects, the one or more MGS peptides have a sequence identity of at least 90, 91, 92, 93, 94, 95, 96, 97, 98, or 99% identity with CH₃CO-YAAWPASGAWT-PEG₁₁-C-NH₂ (SEQ ID NO:12), CH3CO-LQWRRNFGVWARYRL-PEG₁₁-C-NH₂ (SEQ ID NO:22), CH3CO-RGDLATLRQL-PEG₁₁-YC-NH₂ (SEQ ID NO:4), CH3CO-RGDLATLRQL-PEG₁₁-Y-NH₂ (SEQ ID NO:5), CH₃CO-d(Leu)-RGDLATLRQL-PEG₁₁-YC-NH₂ (SEQ ID NO:6), CH₃CO-d(Leu)-RGDLATLRQL-PEG₁₁-Y-NH₂ (SEQ ID NO:7), CH₃CO- KQYATPRVFWT - PEG₁₁-C-NH₂ (SEQ ID NO:16), or CH₃CO-FHAVPQSFYT-PEG₁₁-C-NH₂ (SEQ ID NO:19).

In some aspects, the one or more MGS peptides can be modified by acetylation on the N-terminus. Thus, in some aspects, the one or more MGS peptides can be acetylated. In some aspects, the one or more MGS peptides can be chemically conjugated to a nucleic acid sequence. In some aspects, the chemical conjugate can be polyethylene glycol (PEG). Thus, in some aspects, the one or more MGS peptides can be pegylated. In some aspects, the number of PEG units can be 1, 2, 3, 4, 5, 6, 7, 8, 9, 10, 11, or more. In some aspects, the number of PEG units can be of sufficient length to separate the one or more MGS peptides from the nucleic acid sequence to prevent any steric interference between the one or more MGS peptides and the nucleic acid sequence. For example, disclosed herein are compositions comprising a chemical conjugate, wherein the chemical conjugate is PEG and the PEG comprises eleven PEG units. In an aspect, the one or more MGS peptides comprise SEQ ID NOs: 9 or 10, wherein SEQ ID NOs: 9 or 10 can be acetylated on the N-terminus and can be chemically conjugated to PEG; and the nucleic acid sequence can be covalently attached to PEG.

In an aspect, the one or more MGS peptides disclosed herein can be truncated. In some aspects, the active portion of an MGS peptide can be used in the disclosed compositions. In some aspects, the active portion can be determined using techniques well known in the art, for example Alanine scanning or truncation studies. The active portion of the MGS peptide is the portion that retains its ability to bind to specific cells and target specific locations within the cell. For example, SEQ ID NO:9 can be truncated by at least nine amino acids on the C-terminal end. Thus, an 11-mer of SEQ ID NO: 9, described herein as SEQ ID NO:10 can be used as an MGS peptide. In some aspects, SEQ ID NO:13 can be truncated by about three, four, or five amino acids at the N-terminal and/or C-terminal end.

Disclosed are any of the MGS peptides described in Table 1 or combination thereof. Thus, in some aspects, the one or more MGS peptides can comprise any one of SEQ ID NO: 1-55 or a combination thereof.

In some aspects, the compositions can comprise one or more MGS peptides, for example, in some aspects, the composition can comprise, one, two, three, four or five MGS peptides. In some aspects, the one or more MGS peptides can form a tetrameric scaffold protein. In some aspects, the MGS peptides can be a monomer or a dimer. In some aspects, multiple MGS peptides can be used together, such as, but not limited to, a dimer or trimer. In some aspects, two or more MGS peptides can be used together, wherein the two or more MGS peptides have the same sequence. In some aspects, two or more MGS peptides can be used together, wherein the two or more MGS peptides have different sequence. In some aspects, multiple MGS peptides, such as dimers, comprise two or more of the same MGS sequence. In some aspects, multiple MGS peptides, such as dimers, comprise at least two different MGS sequences.

In some aspects, the one or more MGS peptides localize to one or more intracellular targets. For example, the intracellular target can be, but is not limited to, the lysosome, golgi apparatus, endoplasmic reticulum, cytoplasm, or nucleus. Any subcellular compartment can be targeted.

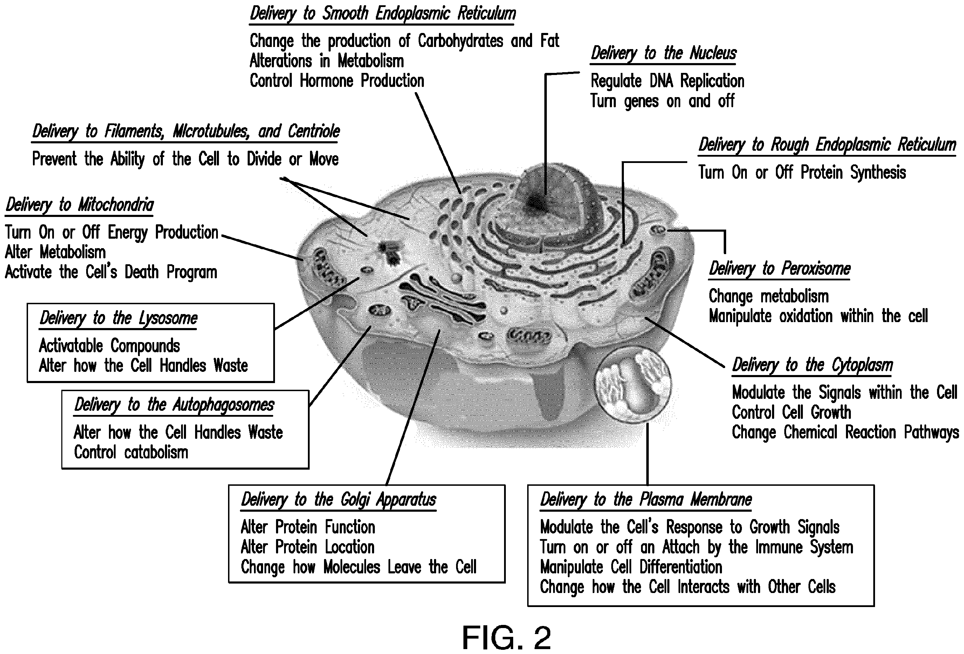

The FOX-Three platform is based on established phage display libraries containing 10⁹ - 10¹² candidate peptide-based MGS members that can be rapidly screened against target cells and subcellular systems to identify specifically targeted MGSs (Fig. 2). Peptides are a well-understood class of biomolecule that are readily synthesized by biological processes and through man-made chemical processes. As a result, the power of the FOX-Three platform is demonstrated in its speed and flexibility. It can be applied to any cell type, regardless of knowledge about the molecular features of that cell, producing a lead MGS in 2-4 weeks. Selected peptide -based MGSs are then readily engineered for optimal performance. Table 2 shows MGSs identified using the FOX-Three Technology.

**Table 2. MGSs identified by FOX-Three Technology**

| **Indication** | | **Cell Type Targeted** | **MSG Code and Cellular Location** | **Cargo Delivered** | |
|---|---|---|---|---|---|
| **Carcinomas and Solid Tumors** | | | MGS_H1299.1_Intracellular | | • Monoclonal Antibodies |
| | | | MGS_H1299.2_Intracellular | | |
| | | | MGS_1299.3_Autophagosomes | | • Small molecule therapeutics (doxorubicin, paclitaxel, DM1, amanitin, auristatin and duocarmycin) |
| | | | MGS_H2009.1_Golgi | | |
| | | | MGS_H2009.2_Lysosome | | |
| | | | MGS_H2009.3_Lysosome | | |
| | | Non-Small Cell Lung Cancer | MGS_H2009.4_Golgi/ER | | |
| | | | MGS_H2009.5_ER/Lysosome | | |
| | | Breast Cancer (including triple negative) | MGS_H1993.1_Lysosome | | |
| | | | MGS_H1993.2_Intracellular | | • Fluorophores |
| | | | MGS_H1993.3_Intracellular | | |
| | | Pancreatic Cancer | MGS_H1993.4_Pre-mitotic cells only | | • Nanoparticles (liposomes, micelles, quantum dots, SPIO) |
| | | Prostate Cancer | | | |
| | | Ovarian Cancer | MGS_H1993.5_Intracellular | | |
| | | Colorectal Cancer | MGS_H1993.6_Intracellular | | • Imaging agents (PET, NIR, MR) |
| | | Liver Carcinoma (additional targets are WIP) | MGS_H460.1_Plasma Membrane | | |
| | | | MGS_HCC15.1_Intracellular | | |
| | | | MGS_HCC15.2_Lysosomal | | • Peptide and proteins |
| | | | MGS_A549.1_Intracellular | | |
| | | | MGS_HCC95.1 | | • Proteinaceous toxins |
| | | | MGS_H1155.1 | | |
| | | | MGS_H1155.2 | | • Antigenic peptides |
| | | | MGS_H666.1 | | • Bead-based capture |
| | | | MGS_MCF7 | | |
| **Lymphoma and Leukemia** | | Lymphoma Cells | MGS_A20.1Plasma Membrane | | • Proteins |
| | | | MGS_A20.2_Plasma Membrane | | • Fluorophores |
| | | | MGS_PCM.1_Plasma Membrane | | • Bead-based capture |
| **Vaccine Development** | | Dendritic Cells | MGSXS52.1_Intacellular | | • Liposomes |
| | | | | | • DNA |
| | | | MGS_XS52.3_Intacellular | | • Protein antigens |
| | | | | | • Fluorophores |
| **Diabetes** | | β-Cells of the Islets of Langerhans | MSG_RII.1 | | • Proteins |
| | | | MGS_RII.2 | | • Radionuclides |
| **Pathogen Infected Cells** | | Mycoplasma Arginine Infected Cells | MGS_MArg.1_Bacterial | | • Fluorescent dyes |
| | | | | | • Bead-based capture |
| **Cardiovascular Disease** | | Cardiomyocytes | MGS_PCM.1_Intracellular | | • DNA |
| | | | | | • Fluorophores |
| | **Intracellular indicates that cellular uptake has been confirmed but the exact intracellular compartment has not been determined.** | | | | |
| | **MGS with no location information indicated that cellular binding has been confirmed but cellular location not determined.** | | | | |
| | **MGSs highlighted in blue have been shown to home to their target cell in an animal model.** | | | | |
| | **The MGSs that target NSCLC also bind to cancers derived form other organ sites such as pancreatic, colorectal, breast, and ovarian cancers.** | | | | |

In some aspects, the one or more MGS peptides can be conjugated to a nucleic acid sequence in any of the ways proteins are commonly conjugated or linked to nucleic acids. In some aspects, the nucleic acid sequence can be conjugated to the one or more MGS peptides via a linker. For example, in some instances the linker can be a peptide linker or a nucleic acid linker. In some aspects the linker can be a cleavable linker. In some aspects, an MGS peptide can be conjugated to a nucleic acid sequence using any known methods, including but not limited to, covalent linkage, bioconjugation chemistry.

In some aspects, a cargo molecule can be conjugated to one or more MGS sequences. IN some aspects, a cargo molecule can be, but is not limited to, a nucleic acid sequence, protein, antibody, peptide, nanoparticle, dye, or small molecule. As described herein, the cargo is a nucleic acid sequence.

### 2. Nucleic acid sequences

In some aspects, a nucleic acid sequence of the disclosed compositions can be DNA, RNA, or a DNA/RNA hybrid. In some aspects, the nucleic acid sequence is an anti-sense oligonucleotide. In some aspects, the nucleic acid sequence is a siRNA or miRNA. In some apsects, the nucleic acid sequence can be any nucleic acid therapeutic.

In some aspects, the nucleic acid sequence targets an intracellular target. In some aspects, the intracellular target can be, but is not limited to, lysosome, golgi apparatus, endoplasmic reticulum, cytoplasm, or nucleus. Any subcellular compartment can be targeted.

In some aspects, the nucleic acid sequence is 10-30 nucleotides in length. In some aspects, the nucleic acid sequence is 10-50 nucleotides in length. In some aspects, the nucleic acid sequence is 10-100 nucleotides in length. In some aspects, the nucleic acid sequence is 8-50 nucleotides in length. In some aspects, the nucleic acid sequence is 5-50 nucleotides in length. In some aspects, the nucleic acid sequence is 5-250 nucleotides in length. In some aspects, the nucleic acid sequence is 5-500 nucleotides in length. In some aspects, the nucleic acid sequence is up to 1 Kb nucleotides in length. In some aspects, the size of the nucleic acid is not a limitation.

In some aspects of the disclosed compositions, the nucleic acid sequence can further comprise a label. For example, the compositions disclosed herein can include detectable labels. In some aspects, the label can be a fluorochrome. Such detectable labels can include, but are not limited to, a tag sequence designed for detection (e.g., purification or localization) of an expressed polypeptide or sequence. Tag sequences include, for example, green fluorescent protein, glutathione S-transferase, polyhistidine, c-myc, hemagglutinin, or Flag^{™} tag, and can be fused with an encoded nucleic acid. Such detectable labels can include, but are not limited to, a fluorescent agent, an enzymatic label, or a radioisotope.

The therapeutically effective amount of the nucleic acid present within the compositions described herein and used in the methods as disclosed herein applied to mammals (e.g., humans) can be determined by one of ordinary skill in the art with consideration of individual differences in age, weight, and other general conditions (as mentioned above). Because the compositions of the present disclosure can be stable in serum and the bloodstream and in some cases more specific, the dosage of the compositions, including any individual component, can be lower (or higher) than an effective dose of any of the individual components when unbound.

In some aspects, a nucleic acid sequence can be T^{m}CAG^{m}CATT^{m}CTAATAG^{m}CAG^{m}C (SEQ ID NO:56). SEQ ID NO:56 is the sequence for MALAT 1.

### 3. Pharmaceutical compositions

In some aspects, the disclosed compositions can be pharmaceutical compositions. For example, in some aspects, disclosed are pharmaceutical compositions comprising a composition comprising a nucleic acid sequence conjugated to one or more MGS peptides and a pharmaceutically acceptable carrier. By "pharmaceutically acceptable" is meant a material or carrier that would be selected to minimize any degradation of the active ingredient and to minimize any adverse side effects in the subject, as would be well known to one of skill in the art. Examples of carriers include dimyristoylphosphatidyl (DMPC), phosphate buffered saline or a multivesicular liposome. For example, PG:PC:Cholesterol:peptide or PC:peptide can be used as carriers in this invention. Other suitable pharmaceutically acceptable carriers and their formulations are described in Remington: The Science and Practice of Pharmacy (19th ed.) ed. A.R. Gennaro, Mack Publishing Company, Easton, PA 1995. Typically, an appropriate amount of pharmaceutically-acceptable salt is used in the formulation to render the formulation isotonic. Other examples of the pharmaceutically-acceptable carrier include, but are not limited to, saline, Ringer's solution and dextrose solution. The pH of the solution can be from about 5 to about 8, or from about 7 to about 7.5. Further carriers include sustained release preparations such as semi-permeable matrices of solid hydrophobic polymers containing the composition, which matrices are in the form of shaped articles, e.g., films, stents (which are implanted in vessels during an angioplasty procedure), liposomes or microparticles. It will be apparent to those persons skilled in the art that certain carriers may be more preferable depending upon, for instance, the route of administration and concentration of composition being administered. These most typically would be standard carriers for administration of drugs to humans, including solutions such as sterile water, saline, and buffered solutions at physiological pH.

Pharmaceutical compositions can also include carriers, thickeners, diluents, buffers, preservatives and the like, as long as the intended activity of the polypeptide, peptide, or conjugate of the invention is not compromised. Pharmaceutical compositions may also include one or more active ingredients (in addition to the composition of the invention) such as antimicrobial agents, anti-inflammatory agents, anesthetics, and the like.

The pharmaceutical compositions as disclosed herein can be prepared for oral or parenteral administration. Pharmaceutical compositions prepared for parenteral administration include those prepared for intravenous (or intra-arterial), intramuscular, subcutaneous, intraperitoneal, transmucosal (e.g., intranasal, intravaginal, or rectal), or transdermal (e.g., topical) administration. Aerosol inhalation can also be used to deliver the fusion proteins. Thus, compositions can be prepared for parenteral administration that includes fusion proteins dissolved or suspended in an acceptable carrier, including but not limited to an aqueous carrier, such as water, buffered water, saline, buffered saline (e.g., PBS), and the like. One or more of the excipients included can help approximate physiological conditions, such as pH adjusting and buffering agents, tonicity adjusting agents, wetting agents, detergents, and the like. Where the compositions include a solid component (as they may for oral administration), one or more of the excipients can act as a binder or filler (e.g., for the formulation of a tablet, a capsule, and the like). Where the compositions are formulated for application to the skin or to a mucosal surface, one or more of the excipients can be a solvent or emulsifier for the formulation of a cream, an ointment, and the like.

The pharmaceutical compositions can be sterile and sterilized by conventional sterilization techniques or sterile filtered. Aqueous solutions can be packaged for use as is, or lyophilized, the lyophilized preparation, which is encompassed by the present disclosure, can be combined with a sterile aqueous carrier prior to administration. The pH of the pharmaceutical compositions typically will be between 3 and 11 (e.g., between about 5 and 9) or between 6 and 8 (e.g., between about 7 and 8). The resulting compositions in solid form can be packaged in multiple single dose units, each containing a fixed amount of the above-mentioned agent or agents, such as in a sealed package of tablets or capsules. The composition in solid form can also be packaged in a container for a flexible quantity, such as in a squeezable tube designed for a topically applicable cream or ointment.

The pharmaceutical compositions described above can be formulated to include a therapeutically effective amount of a composition disclosed herein. In some aspects, therapeutic administration encompasses prophylactic applications. Based on genetic testing and other prognostic methods, a physician in consultation with their patient can choose a prophylactic administration where the patient has a clinically determined predisposition or increased susceptibility (in some cases, a greatly increased susceptibility) to one or more autoimmune diseases or where the patient has a clinically determined predisposition or increased susceptibility (in some cases, a greatly increased susceptibility) to cancer.

The pharmaceutical compositions described herein can be administered to the subject (e.g., a human subject or human patient) in an amount sufficient to delay, reduce, or preferably prevent the onset of clinical disease. Accordingly, in some aspects, the subject is a human subject. In therapeutic applications, compositions are administered to a subject (e.g., a human subject) already with or diagnosed with an autoimmune disease in an amount sufficient to at least partially improve a sign or symptom or to inhibit the progression of (and preferably arrest) the symptoms of the condition, its complications, and consequences. An amount adequate to accomplish this is defined as a "therapeutically effective amount." A therapeutically effective amount of a pharmaceutical composition can be an amount that achieves a cure, but that outcome is only one among several that can be achieved. As noted, a therapeutically effective amount includes amounts that provide a treatment in which the onset or progression of the cancer is delayed, hindered, or prevented, or the autoimmune disease or a symptom of the autoimmune disease is ameliorated. One or more of the symptoms can be less severe. Recovery can be accelerated in an individual who has been treated.

The total effective amount of the conjugates in the pharmaceutical compositions disclosed herein can be administered to a mammal as a single dose, either as a bolus or by infusion over a relatively short period of time, or can be administered using a fractionated treatment protocol in which multiple doses are administered over a more prolonged period of time (e.g., a dose every 4-6, 8-12, 14-16, or 18-24 hours, or every 2-4 days, 1-2 weeks, or once a month). Alternatively, continuous intravenous infusions sufficient to maintain therapeutically effective concentrations in the blood are also within the scope of the present disclosure.

### C. Methods

Disclosed are methods of using the disclosed compositions. Any of the compositions disclosed herein can be used in the methods disclosed herein.

Disclosed are methods of decreasing gene expression of a gene of interest comprising administering to a cell a composition comprising a nucleic acid sequence conjugated to one or more MGS peptides, wherein the nucleic acid sequence binds to the RNA transcribed from the gene of interest, the gene of interest or a sequence upstream of the gene of interest. In some aspects, a nucleic acid sequence can be any therapeutic nucleic acid sequence, such as, but not limited to, antisense oligonucleotides, aptamers, and small interfering RNAs. In some aspects, decreasing gene expression is determined by measure protein levels. Thus, a decrease in gene expression results in a decrease in protein levels.

Disclosed are methods of increasing gene expression of a gene of interest comprising administering to a cell a composition comprising nucleic acid sequence conjugated to one or more MGS peptides, wherein the nucleic acid sequence binds to the gene of interest or a sequence upstream of the gene of interest.

Thus, disclosed are methods of modifying gene expression of a gene of interest comprising administering to a cell a composition comprising a nucleic acid sequence conjugated to one or more MGS peptides, wherein the nucleic acid sequence binds to the RNA transcribed from the gene of interest, the gene of interest or a sequence upstream of the gene of interest. In some aspects, modifying gene expression of a gene of interest comprises increasing or decreasing gene expression of a gene of interest.

In some aspects, the sequence upstream of a gene of interest can be a region of the DNA responsible for regulating gene expression of the gene interest. In some aspects, binding to a sequence upstream of the gene of interest can increase or decrease gene expression of the gene of interest.

Disclosed are methods of targeting a gene of interest in an intracellular target comprising administering to a cell a composition comprising a nucleic acid sequence conjugated to one or more MGS peptides, wherein the MGS peptide targets the intracellular target, wherein the nucleic acid sequence binds to the RNA transcribed from the gene interest in an intracellular target. In some aspects, the intracellular target can be, but is not limited to, a lysosome, Golgi apparatus, endoplasmic reticulum, cytoplasm, or nucleus.

In some aspects of the disclosed methods, a gene of interest can be any gene whose expression or over expression is harmful to the cell or involved in a disease process. For example, a gene of interest can be a gene that is over or under expressed in one of the disclosed intracellular targets (e.g. lysosome, Golgi apparatus, endoplasmic reticulum, cytoplasm, or nucleus) thus causing disease. In some aspects, a gene of interest can be, but is not limited, KRAS or MYC.

Disclosed are methods of treating a subject in need thereof comprising administering to the subject in need thereof an effective amount of a nucleic acid sequence conjugated to one or more MGS peptides, wherein the MGS peptide targets an intracellular target involved in a disease process. In some aspects, the subject in need thereof has an infectious disease, cancer, diabetes, a neurological or neurodegenerative disease, a genetically inherited disease, a lysosomal disease, a mitochondrial disease, or been exposed to a bioterrorism agent.

In some aspects, the disclosed methods can use one or more of the MGS peptides described in Table 1.

In some aspects, the intracellular target can be, but is not limited to, a lysosome, Golgi apparatus, endoplasmic reticulum, cytoplasm, or nucleus.

In some aspects, the nucleic acid sequence binds to RNA transcribed from a gene of interest inside the intracellular target. In some aspects, the binding of the nucleic acid sequence to a RNA transcribed from a gene of interest results in a decrease in expression of the gene of interest. In some aspects, the nucleic acid sequence binds to the gene of interest inside the intracellular target. In some aspects, the nucleic acid sequence binds to a sequence upstream of a gene of interest inside the intracellular target. In some aspects, the sequence upstream of a gene of interest can be a region of the DNA responsible for regulating gene expression of the gene interest. In some aspects, binding to a sequence upstream of the gene of interest can increase or decrease gene expression of the gene of interest.

In some aspects, the gene of interest is any gene whose under expression or over expression is involved in the disease process. For example, a gene of interest can be a gene that is over or under expressed in one of the disclosed intracellular targets (e.g. lysosome, Golgi apparatus, endoplasmic reticulum, cytoplasm, or nucleus) thus causing disease. In some aspects, a gene of interest can be, but is not limited, KRAS or MYC.

In some aspects, the disclosed methods of treating can further comprise administering a one or more additional therapies. In some aspects, the disclosed compositions can be administered alone or in combination with other biologically active agents. In some aspects, the disclosed compositions can be formulated alone or in combination with the one or more additional therapies (e.g. biologically active agent) into compositions suitable for administration to a subject. In an aspect, methods directed to treating subjects with cancer or at risk for developing cancer, the compositions disclosed herein can be combined with, for example, therapeutically effective amount of radiation therapy, immunotherapy or chemotherapy or a combination thereof. The combined therapy can be administered as a co-formulation, or separately. When administered separately, the combined therapy can be administered simultaneously or sequentially. The formulations can be made using methods routine in the art.

### D. Vectors

Disclosed are vectors comprising the nucleic acid sequence that encodes for one or more of the disclosed compositions. In some aspects, the vector comprises only a nucleic acid sequence capable of encoding one or more of the disclosed MGS peptides.

### E. Kits

The materials described above as well as other materials can be packaged together in any suitable combination as a kit useful for performing, or aiding in the performance of, the disclosed method. It is useful if the kit components in a given kit are designed and adapted for use together in the disclosed method. For example disclosed are kits comprising one or more of the disclosed compositions.

In some aspects, the disclosed kits can comprise one or more of the disclosed MGS peptides and/or one or more of the disclosed nucleic acid sequences.

### Examples

The FOX-Three Platform has already been validated for targeting of cells and subcellular components. A number of validated MGSs are in various stages of development (Table 1) for a variety of cell targeting systems. Methods of conjugating a variety of payloads to the peptide MGSs have been established without disrupting their targeting ability. Selected MGSs have been demonstrated to deliver drugs, imaging agents, nanoparticles, DNA, and proteins to target cells in culture and in animal models. The FOX-Three screening process was recently refined to include subcellular location targeting as a selection criteria. MGSs have been isolated that accumulate in lysosomes, autophagosomes, and Golgi inside cancer cells, as well as target the plasma membrane. The therapeutic efficacy of MGSs has been demonstrated to be dependent on delivery to the correct subcellular location.

With the wealth of cell types, disease states, intracellular organelles, and payloads that can be delivered, the potential of the FOX-Three platform is vast. A robust platform for creating a 'toolbox' of optimized MGSs can be developed that can deliver a focused against select disease cell types and subcellular components. To do this the FOX-Three platform can be used to expand the number of subcellular organelles that can be targeted. A set of experiments with quantitative milestones can be performed.

In some aspects, synthesis, characterization, and optimization of 3 MGSs that target a non-small lung cancer cell line and accumulate in the lysosome, Golgi, and mitochondria can be studied.

Furthermore, the subcellular locations can be expanded to include lysosome, Golgi, mitochondria, Endoplasmic reticulum, cytoplasm, and nucleus.

The FOX-Three discovers and offers the rapid development of intracellular targeting human therapeutic antibodies in unprecedented speed to create extremely safe and rapid responses to emerging threats.

The developed FOX-Three MGS toolbox can deliver a variety of other payloads. While the delivery of has been a main focus, it is important to note that MGSs can deliver many different payloads and have other clinical applications (Table 1). MGSs can deliver small molecule drugs, imaging agents, nanoparticles, DNA, radionuclides and other proteins. MGSs can be employed for early detection of disease and as companion diagnostics. Early disease detection is often the major determinant of clinical outcome. Companion diagnostics can follow the response to treatment, allowing for rapid changes in treatment, as necessary, saving time and costs. The MGS-therapeutic can be used for targeted therapeutics (small molecule, nucleic acid. Synthetic macromolecules such as polymers, and protein) for a wide variety of disease states, in vitro and in vivo diagnostics, personalized therapies for cancer, intracellular nanoparticle delivery, and innovative immunotherapies.

**Table 3. MGS sequences**

| **Original MGS Name** | **Selected Sequence** | **Optimized Sequence** | **Optimal Valency** | **Cellular Location** | **In Vivo Targeting** |
|---|---|---|---|---|---|
| **Carcinomas and Solid Tumors** | | | | | |
| MGS_1299.2 | YAAWPASGA WTGTAPC SAG T (SEQ ID NO:9) | SRI_MGS2 V4 CH3CO- YAAWPASGAWT-PEGII-C-NH2 (SEQ ID NO:12) | Dimer | Lysosome | Yes |
| MGS_1299.3 | LQWRRDDNV HNFGVWARY RL (SEQ ID NO:20) | SRI_MGSS V2 CH3CO- LQWRRNFGVWARYRL-PEG11-C- NH2 (SEQ ID NO:22) | Dimer | Autophagas ome | Yes |
| MGS_H2009.1 | RGDLATLRQ L AQEDGVVGV R (SEQ ID NO:1) | SRI_MGS1 V4 CH3CO- RGDLATLRQL-PEG11-YC-NH2 (SEQ ID NO:4) SRI_MGS1 V5 CH3CO-d(Leu)- RGDLATLRQL-PEG11-YC-NH2 (SEQ ID NO:6) | Dimer Dimer | Golgi | Yes |
| MGS_HCC15.1 | ATEPRKQYAT PRVFWTDAPG (SEQ ID NO:13) | SRI_MGS3_V4 CH3CO- KQYATPRVFWT-PEGII-C-NH2 (SEQ ID NO:16) | Dimer | Lysosome | Yes |
| MGS_HCC15.2 | FHAVPQSFYT AP  (SEQ ID NO:17) | SRI_MGS4 V8 CH3CO- FHAVPQSFYT -PEGII-C-NH2 (SEQ ID NO:19) | Monomer Dimer | Lysosome | Yes |
| MGS_H2009.2 | EHPWFNMWS WATQVQE (SEQ ID NO:30) | | | Lysosome | |

Below is an example of the structure of the multimerization core where R represents an MGS conjugated through a sulfhydryl group of the cysteine. R' is the attachment site for the cargo (e.g. MAb, protein, other peptides, drugs, nanoparticles, imaging agents, nucleic acids, or dyes). Attachment can occur through maleimide chemistry, click chemistry, amide chemistry, and through hydrazones.

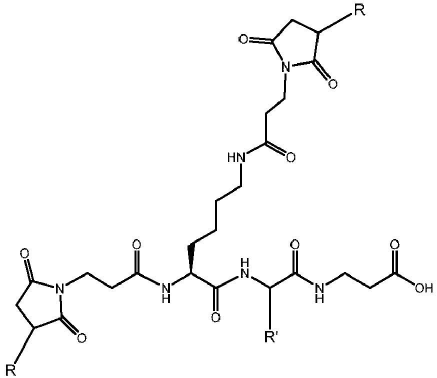

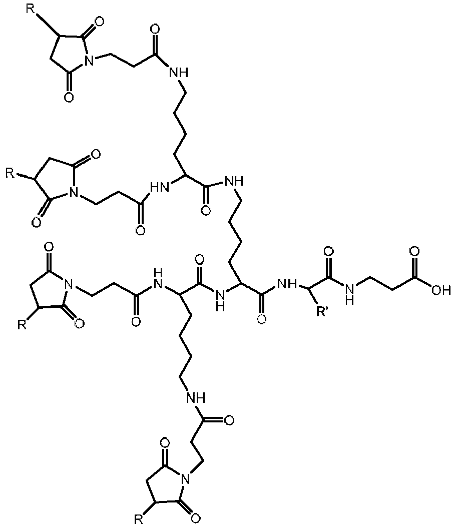

Dimers have also been synthesized using linear peptide chemistry (no maleimide group). Below is an example of a structure of a dimer prepared using linear peptide chemistry (no maleimide group) where R represents an MGS and R' represents the attachment site for the cargo (e.g. MAb, protein, other peptides, drugs, nanoparticles, imaging agents, nucleic acids, or dyes).

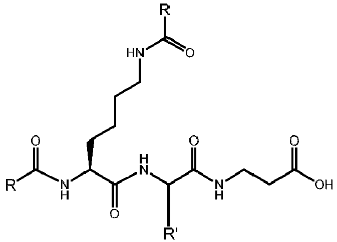

### 1. Example 1: Effective dose, stoichiometry, and rate of uptake for MGS 2.

Table 4 below shows EC50 values for an MGS for various cell lines. As the numbers indicate, the molecules internalized is between approximately 50,000 to approximately 125,000 and having a half-life of between 11 and 37 minutes.

**Table 4: SRI_MGS2_V4 profile**

| | | SRI_MGS2_V4 | | | | |
|---|---|---|---|---|---|---|
| Cell line | | EC₅₀ (nM) | ¹Saturation (molecules) | ²Molecules Internalized, 6H | t_{1/2} | ³Specificity |
| H2009 Cells | | 0.72 | 68300 ± 3030 | 49500 ± 2230 | 13 | 560 |
| H358 Cells | | 0.89 | 99900 ± 9640 | 97100 ± 4830 | 11 | 1100 |
| H1993 Cells | | 1.2 | 141600 ± 28200 | 134300 ± 6300 | 12 | 2300 |
| H1299 Cells | | 1.4 | 112800 ± 15500 | 125500 ± 5430 | 37 | 1400 |
| | **1. Average number of MGS molecules internalized per cell at saturation concentration at 1 H.** | | | | | |
| | 2. Average number of molecules internalized at 6 H with constant incubation of 20 nM MGS. | | | | | |
| | 3. Ratio of MGS uptake on indicated cancer cell line vs. a non-transformed, immortalized normal human bronchial epithelial cell line (HBEC). | | | | | |

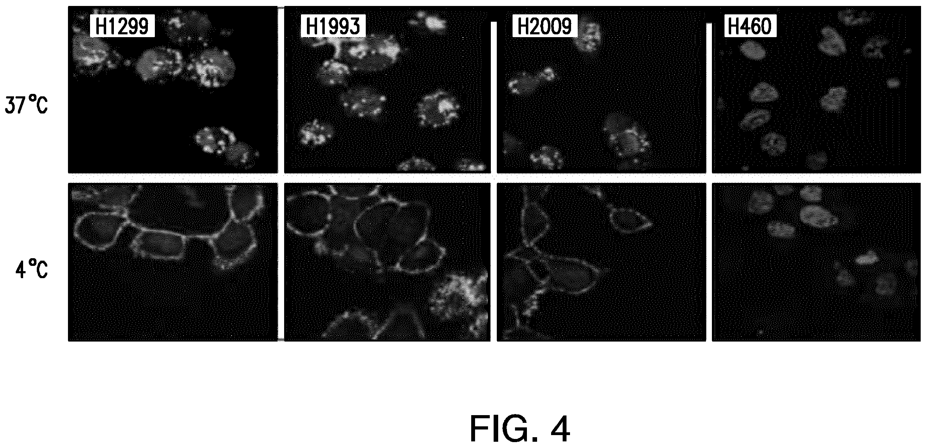

FIG. 3 shows internalization for four different cell types at two different temperatures and with a control with no MGS. Furthermore, FIG. 4 shows presence of the fluorophore internalized into the cells at two different temperatures.

### 2. Example 2: MGS and nucleic acid as cargo

The concept of MGS was applied to nucleic acids. Nucleic acids make good candidates for therapy because they are known to regulate gene expression, but nucleic acid therapies have been slower to advance as therapeutics due to their poor pharmacological properties (e.g. larger size, highly charged, and fast clearance from the body). If MGS could be applied to nucleic acids and aid with the internalization of therapeutic nucleic acids, this would open the door to using nucleic acids in new therapies.

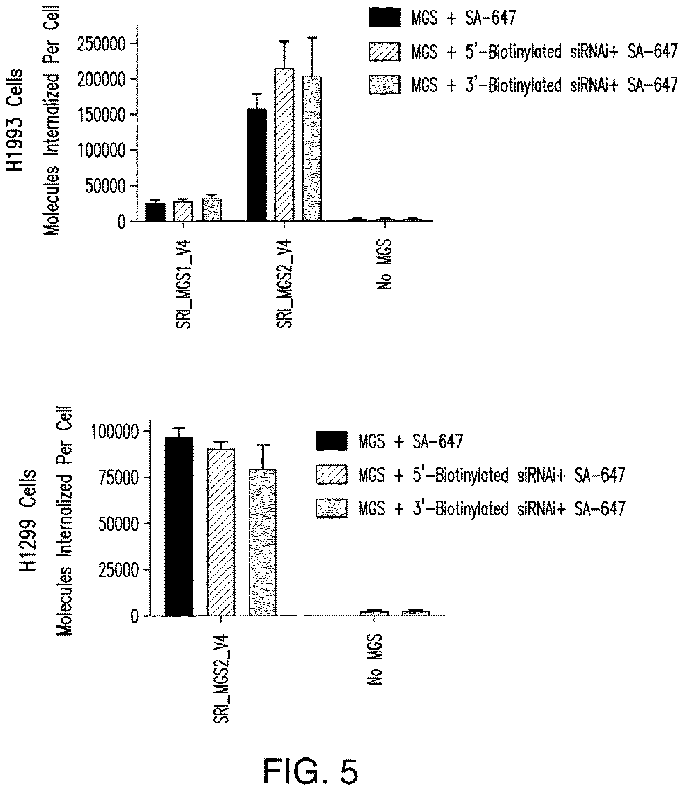

With this objective, novel MGSs were applied to facilitate cell-specific delivery of siRNA. As previously mentioned, these MGSs have the unique ability to bind to specific epithelial-derived cancer cell types, and upon binding, triggers rapid internalization and trafficking to specific subcellular locations. The chemically optimized MGSs have 1-2 nM affinities for their cellular targets, serum stability greater than 48 hours, and approximately 50-1000-fold specificity for targeted cancer cells over normal control cells. Target cells internalize these peptidic ligands rapidly, reaching intracellular concentrations up to 1.5 µM with t_{1/2} of approximately 10-30 minutes. FIG. 5 shows a comparison of siRNA into the cells with no MGS and with MGS biotinylated at either the 5' or 3' end of the siRNA. Internalization experiments were first performed using H1993 cells. For this set of experiments, the group of bars at the far right are 5'-Biotinylated siRNA+SA-647 and 3'-Biotinylated siRNA+SA-647 without MGS. The middle and far left bar groups show SA-647, 5'-Biotinylated siRNA+SA-647, and 3'-Biotinylated siRNA+SA-647 conjugated to a first MGS (SRI_MGS1_V4, right) and a second MGS (SRI_MGS2_V4, middle). As can be seen, no siRNA was internalized into the H1993 cells by themselves. With conjugating of the three different forms of siRNA to SRI_MGS1_V4, data shows approximately 25000 molecules internalized per cell for all three forms of siRNA, while conjugating of the three different forms of siRNA to SRI_MGS2_V4, showed even better internalization of between approximately 150,000 to 210,000 molecules internalized per cell. Similar results were seen for experiments using H1299 cells, where the internalization was close to zero for the siRNA forms with no MGS and approximate internalization of approximately 80,000 to approximately 95,000 for siRNA forms conjugated to SRI_MGS2_V4.

In summary, this technology has the ability to rapidly generate MGS- that target pathogen infected cells, improving treatment for existing as well as emerging pathogens in weeks versus years. Current technologies are too slow to develop targeting treatments for evolving viruses.

This technology opens up new classes of therapeutics for the treatment of numerous diseases by delivering previously cell-impermeable compounds enabling an entirely new armory for protecting the warfighter

This technology provides the ability to detect and neutralize latent intracellular viral infections that may otherwise reactivate or spread/re-spread throughout a community.

This technology can rapidly develop MGSs that can be used in diagnostics and sensor technologies for biological readouts.

It will be apparent to those skilled in the art that various modifications and variations can be made in the present invention without departing from the scope or spirit of the invention. Other aspects of the invention will be apparent to those skilled in the art from consideration of the specification and practice of the invention disclosed herein. It is intended that the specification and examples be considered as exemplary only, with a true scope and spirit of the invention being indicated by the following claims.

Those skilled in the art will recognize, or be able to ascertain using no more than routine experimentation, many equivalents to the specific embodiments of the method and compositions described herein. Such equivalents are intended to be encompassed by the following claims.
Various preferred features and embodiments of the present invention will now be described with reference to the following numbered paragraphs.
1. A composition comprising a nucleic acid sequence conjugated to one or more molecular guidance system (MGS) peptides.
2. The composition of paragraph 1, wherein the nucleic acid sequence is an anti-sense oligonucleotide.
3. The composition of paragraph 1 or 2, wherein the nucleic acid sequence targets an intracellular target.
4. The composition of any one of paragraphs 1-3, wherein the one or more MGS peptides comprise sequence:
   YAAWPASGAWTGTAPCSAGT (SEQ ID NO:9);
   LQWRRDDNVHNFGVWARYRL (SEQ ID NO:20);
   RGDLATLRQLAQEDGVVGVR (SEQ ID NO: 1);
   ATEPRKQYATPRVFWTDAPG (SEQ ID NO: 13);
   FHAVPQSFYTAP (SEQ ID NO: 17) ;
   EHPWFNMWSWATQVQE (SEQ ID NO:30); or a combination thereof.
5. The composition of any one of paragraphs 1-3, wherein the one or more MGS peptides have a sequence identity of at least 95% to 99% identity with
   YAAWPASGAWTGTAPCSAGT (SEQ ID NO:9),
   LQWRRDDNVHNFGVWARYRL (SEQ ID NO:20),
   RGDLATLRQLAQEDGVVGVR (SEQ ID NO:1), ATEPRKQYATPRVFWTDAPG (SEQ ID NO: 13), FHAVPQSFYTAP (SEQ ID NO: 17), EHPWFNMWSWATQVQE (SEQ ID NO:30); or a combination thereof.
6. The composition of any one of paragraphs 1-5, wherein the one or more MGS peptides are acetylated.
7. The composition of any one of paragraphs 1-6, wherein the one or more MGS peptides are pegylated.
8. The composition of any one of paragraphs 6-7 wherein the one or more MGS peptides comprise sequence:
   CH₃CO-YAAWPASGAWT-PEG₁₁-C-NH2 (SEQ ID NO:12);
   CH₃CO-LQWRRNFGVWARYRL-PEG₁₁-C-NH2 (SEQ ID NO:22);
   CH₃CO-RGDLATLRQL-PEG₁₁-YC-NH2 (SEQ ID NO:4);
   CH₃CO-d(Leu)-RGDLATLRQL-PEG₁₁-YC-NH₂ (SEQ ID NO:6);
   CH₃CO-d(Leu)-RGDLATLRQL-PEG₁₁-Y-NH₂ (SEQ ID NO:7);
   CH₃CO- KQYATPRVFWT-PEG₁₁-C-NH2 (SEQ ID NO:16);
   CH₃CO-FHAVPQSFYT-PEG₁₁-C-NH2 (SEQ ID NO:19); or a combination thereof.
9. The composition of any one of paragraphs 6-7, wherein the one or more MGS peptides have a sequence identity of at least 95% to 99% identity with CH3CO-YAAWPASGAWT-PEG11-C-NH2 (SEQ ID NO:12); CH3CO-LQWRRNFGVWARYRL-PEG11-C-NH2 (SEQ ID NO:22); CH3CO-RGDLATLRQL-PEG11-YC-NH2 (SEQ ID NO:4); CH3CO-d(Leu)-RGDLATLRQL-PEG11-YC-NH2 (SEQ ID NO:6); CH3CO-d(Leu)-RGDLATLRQL-PEG11-Y-NH2 (SEQ ID NO:7); CH3CO- KQYATPRVFWT-PEG11-C-NH2 (SEQ ID NO:16); CH3CO-FHAVPQSFYT-PEG11-C-NH2 (SEQ ID NO:19); or a combination thereof.
10. The composition of any one of paragraphs 1-9, wherein the nucleic acid sequence is conjugated to one or more MGS peptide via a linker.
11. The composition of any one of paragraphs 1-10, wherein the nucleic acid sequence comprises a label.
12. The composition of paragraph 11, wherein the label is a fluorochrome.
13. The composition of any one of paragraphs 1-12, wherein the nucleic acid sequence is 10-30 nucleotides in length.
14. The composition of any one of paragraphs 1-13, wherein the nucleic acid sequence is DNA or RNA.
15. A method of decreasing gene expression of a gene of interest comprising administering to a cell a nucleic acid sequence conjugated to one or more MGS peptides, wherein the nucleic acid sequence binds to the RNA transcribed from the gene of interest.
16. A method of targeting a gene of interest in an intracellular target comprising administering to a cell a nucleic acid sequence conjugated to one or more MGS peptides, wherein the MGS peptide targets the intracellular target, wherein the nucleic acid sequence binds to the RNA transcribed from the gene interest in an intracellular target.
17. The method of paragraph 16, wherein the intracellular target is a lysosome, Golgi apparatus, endoplasmic reticulum, cytoplasm, or nucleus.
18. The method of paragraph 16 or 17, wherein the gene of interest is any gene whose expression or over expression is harmful to the cell.
19. A method of treating a subject in need thereof comprising administering to the subject in need thereof an effective amount of a nucleic acid sequence conjugated to one or more MGS peptides, wherein the MGS peptide targets an intracellular target involved in a disease process.
20. The method of paragraph 19, wherein the intracellular target is a lysosome, Golgi apparatus, endoplasmic reticulum, cytoplasm, or nucleus.
21. The method of paragraph 19 or 20, wherein the nucleic acid sequence binds to RNA transcribed from a gene of interest inside the intracellular target.
22. The method of paragraph 21, wherein the gene of interest is any gene whose expression or over expression is involved in the disease process.
23. The method of paragraph 21 or 22, wherein the binding of the nucleic acid sequence to the RNA transcribed from the gene of interest results in a decrease in expression of the gene of interest.
24. The methods of any one of paragraphs 15-23, wherein the MGS peptide is one or more of the MGS peptides described in Table 1.

## Claims

1. A composition comprising a nucleic acid sequence conjugated to a molecular guidance system (MGS) peptide, wherein the MGS peptide is an amino acid sequence selected from:
d(Leu)-RGDLATLRQL (SEQ ID NO: 2), CH₃CO-d(Leu)-RGDLATLRQL (SEQ ID NO: 3), CH₃CO-RGDLATLRQL-PEG₁₁-YC-NH₂ (SEQ ID NO: 4), CH₃CO-RGDLATLRQL-PEG₁₁-Y-NH₂ (SEQ ID NO: 5), CH₃CO-d(Leu)-RGDLATLRQL-PEG₁₁-YC-NH₂ (SEQ ID NO: 6), CH₃CO-d(Leu)-RGDLATLRQL-PEG₁₁-Y-NH₂ (SEQ ID NO: 7), CH₃CO-RGDLATLRQL (SEQ ID NO: 8), YAAWPASGAWT (SEQ ID NO: 10), CHsCO-YAAWPASGAWT (SEQ ID NO: 11), CH₃CO-YAAWPASGAWT-PEG₁₁-C-NH₂ (SEQ ID NO: 12), KQYATPRVFWT (SEQ ID NO: 14), CH₃CO-KQYATPRVFWT (SEQ ID NO: 15), CH₃CO-KQYATPRVFWT-PEG₁₁-C-NH₂ (SEQ ID NO: 16), CH₃CO-FHAVPQSFYT (SEQ ID NO: 18), CH₃CO-FHAVPQSFYT-PEG₁₁-C-NH₂ (SEQ ID NO: 19), LQWRRNFGVWARYRL (SEQ ID NO: 21), CH₃CO-LQWRRNFGVWARYRL-PEG₁₁-C-NH₂ (SEQ ID NO: 22), EHPWFNMWSWATQVQE (SEQ ID NO: 30), and EHPWFNMWSWATQVQEKKK (SEQ ID NO: 31).

2. The composition of claim 1, wherein the nucleic acid sequence is an anti-sense oligonucleotide.

3. The composition of claim 1 or claim 2, wherein the nucleic acid sequence targets an intracellular target.

4. The composition of claim 3, wherein the intracellular target is a lysosome, Golgi apparatus, endoplasmic reticulum, cytoplasm, or nucleus.

5. The composition of any one of claims 1-4, wherein the nucleic acid sequence is conjugated to the MGS peptide via a linker.

6. The composition of any one of claims 1-5, wherein the nucleic acid sequence comprises a label, optionally a fluorochrome.

7. The composition of any one of claims 1-6, wherein the nucleic acid sequence is 10-30 nucleotides in length.

8. The composition of any one of claims 1-7, wherein the nucleic acid sequence is DNA or RNA.

9. An *in vitro* or *ex vivo* method comprising administering to a cell the composition of any of claims 1-8.

10. The method of claim 9, further comprising the nucleic acid sequence binding to RNA transcribed from a gene of interest, wherein the binding of the nucleic acid sequence to the RNA transcribed from the gene of interest results in a decrease in expression of the gene of interest.

11. The method of claim 9, wherein the MGS peptide targets an intracellular target, the method further comprising the nucleic acid sequence binding to RNA transcribed from a gene of interest in the intracellular target.

12. The method of any of claims 10-11, wherein the gene of interest is any gene whose expression or over expression is harmful to the cell.

13. A composition of any one of claims 1-8, wherein the MGS peptide targets an intracellular target involved in a disease process, for use in therapy.

14. The composition for use according to claim 13, wherein the nucleic acid sequence binds to RNA transcribed from a gene of interest inside the intracellular target, optionally wherein the binding of the nucleic acid sequence to the RNA transcribed from the gene of interest results in a decrease in expression of the gene of interest.

15. A molecular guidance system (MGS) peptide of amino acid sequence selected from:
CH₃CO-RGDLATLRQL-PEG₁₁-Y-NH₂ (SEQ ID NO: 5), CH₃CO-d(Leu)-RGDLATLRQL-PEG₁₁-Y-NH₂ (SEQ ID NO: 7), CH₃CO-RGDLATLRQL (SEQ ID NO: 8), and CH₃CO-KQYATPRVFWT-PEG₁₁-C-NH₂ (SEQ ID NO: 16).
